# EUROPÄISCHE PATENTSCHRIFT

(11) **EP 3 233 393 B1**
(45) Veröffentlichungstag und Bekanntmachung des Hinweises auf die Patenterteilung: **01.05.2019**
(21) Anmeldenummer: 15805152.4
(22) Anmeldetag: 03.12.2015
(51) Int. Cl.: B25J 9/16, G05B 19/409, A61B 34/30

(54) **VERFAHREN ZUM SICHEREN EIN- UND AUSKOPPELN EINES EINGABEGERÄTES**
METHOD FOR SAFE COUPLING AND DECOUPLING OF AN INPUT DEVICE
PROCÉDÉ PERMETTANT DE COUPLER ET DE DÉCOUPLER DE MANIÈRE FIABLE UN APPAREIL D'ENTRÉE DE DONNÉES

(30) Priorität: 17.12.2014 DE 102014226239
(43) Veröffentlichungstag der Anmeldung: 25.10.2017
(73) Patentinhaber: KUKA Deutschland GmbH, 86165 Augsburg (DE)
(72) Erfinder: KELLER, Henrik, 86153 Augsburg (DE); FINKE, Markus, 86438 Kissing (DE); GROCH, Anja, 80798 München (DE); HEINIG, Maximilian, 80639 München (DE); MARTINEZ, Horacio, 81669 München (DE); MEISSNER, Christian, 04275 Leipzig (DE); MILLER, Mario, 86316 Friedberg (DE); MÖNNICH, Holger, 86316 Friedberg (DE); NEFF, Thomas, 86163 Augsburg (DE); REICHL, Tobias, 80939 München (DE); SAUTHOFF, Nina, 37574 Einbeck (DE); SHAHIN, Osama, 86153 Augsburg (DE); THILMANN, Oliver, 86159 Augsburg (DE); WEGENER, Olaf, 44143 Dortmund (DE)
(74) Vertreter: Mader, Joachim
(86) Internationale Anmeldenummer: PCT/EP2015/078503
(87) Internationale Veröffentlichungsnummer: WO 2016/096456

(56) Entgegenhaltungen:
- EP-A1- 2 113 344
- EP-A2- 2 123 407
- DE-A1-102012 206 350
- US-A1- 2010 198 402
- US-A1- 2012 245 595

## Beschreibung

### 1. Gebiet der Erfindung

Die Erfindung betrifft ein Verfahren zum sicheren Einkoppeln und Auskoppeln eines Eingabegerätes mit einem Manipulator, wobei das Eingabegerät zur Steuerung des Manipulators eingerichtet ist und im eingekoppelten Zustand den Manipulator steuern kann. Das Verfahren und der so gesteuerte Manipulator können beispielsweise für einen minimalinvasiven laparoskopischen Eingriff verwendet werden.

### 2. Hintergrund

Für viele Anwendungen in der Robotik, insbesondere im Bereich der Telemanipulation, werden Eingabegeräte benötigt, die translatorisch oder rotatorisch bewegt werden können. Bei der Telemanipulation werden Bewegungen des Eingabegerätes erfasst und in translatorische und/oder rotatorische Bewegungen eines oder mehrerer Manipulatoren umgesetzt.

Ein Manipulator ist gemäß DIN EN ISO 8373 ein automatisch gesteuerter, frei programmierbaren Mehrzweck-Manipulator, der in drei oder mehr Achsen programmierbar ist und zur Verwendung beispielsweise in der Automatisierungstechnik entweder an einem festen Ort oder beweglich angeordnet sein kann. Ein Manipulator führt Greifer, Werkzeuge oder Werkstücke. Manipulatoren werden auch im medizinischen Bereich eingesetzt, beispielsweise in der minimalinvasiven Chirurgie. Dort kann der Manipulator ein laparoskopisches Instrument führen und sehr kleine Strukturen bearbeiten, oder durch Halten und Führen eines Endoskops die minimalinvasive Bildgebung ermöglichen.

Abhängig von der Anwendung kann es notwendig sein, zu bestimmten Zeitpunkten oder aufgrund von Ereignissen, wie zum Beispiel aufgrund eines Signals eines Totmannschalters oder des Verlassens des Arbeitsraums des Manipulators, eine Benutzereingabe nicht mehr unmittelbar in eine Manipulatorbewegung umzusetzen. Eine solche Unterbrechung der Umsetzung von Benutzereingaben in Manipulatorbewegungen wird im Folgenden "Auskoppeln" genannt. Entsprechend kann es nach einem Auskoppeln notwendig sein, zu einem bestimmten Zeitpunkt oder aufgrund eines bestimmten Ereignisses die Umsetzung von Benutzereingaben in Manipulatorbewegungen wieder zuzulassen. Dies wird im Folgenden "Einkoppeln" genannt.

Wird während des ausgekoppelten Zustandes das Eingabegerät oder ein Arm des Manipulators bewegt, so sind Position und Orientierung (Pose) des Eingabegeräts und die entsprechende, kommandierte Position und Orientierung (Pose) des Manipulators nicht mehr synchron zueinander. In diesem Fall hätte ein erneutes Einkoppeln eine unkontrollierte Bewegung des Manipulators in die vom Eingabegerät aktuell kommandierte Position und Orientierung (Pose) zur Folge. Ein solches unkontrolliertes Bewegen eines Manipulators ist für einen sicheren Betrieb unerwünscht oder unzulässig.

Die Position ist durch Angaben bezüglich aller drei translatorischen Freiheitsgrade bestimmt und ist somit der Ort eines Punktes im Raum. Die Orientierung ist durch Angaben bezüglich aller drei rotatorischen Freiheitsgrade bestimmt. Als Pose wird die Kombination von Position und Orientierung eines Objektes bezeichnet. Zur Bestimmung der Pose eines Punktes, sind dem Punkt drei Raumachsen zugeordnet. Somit können alle sechs möglichen Freiheitsgrade des Punktes bestimmt werden.

Aus der Druckschrift EP 1 876 505 A1 sind Eingabegeräte bekannt, die mit aktiven Achsen mit Motoren oder Bremsen ausgestattet sind, um ein Bewegen des Eingabegeräts im ausgekoppelten Zustand zu verhindern. Dadurch wird das Eingabegerät auch nach dem Auskoppeln in der gegenwärtigen Pose gehalten. Solche Eingabegeräte mit aktiven Achsen sind jedoch teuer, technisch aufwändig und fehleranfällig.

Die DE 10 2012 206350 A1 zeigt ein Verfahren zur Umsetzung von Benutzereingaben in Manipulatorbewegungen, wobei der Arbeitsraum durch darin befindliche Objekte begrenzt wird und der TCP des Manipulators den begrenzten Arbeitsraum nicht verlassen darf.

In der US 2012/245595 A1 ist ein Verfahren zur Umsetzung von Benutzereingaben in Manipulatorbewegungen mit haptischer Rückmeldung mit Kollisionsüberwachung gezeigt.

Aus der US 2010/198402 A1 ist ein Verfahren zur Umsetzung von Benutzereingaben in Manipulatorbewegungen bekannt, wobei eine vorbestimmte Bewegung durchgeführt wird und diese durch eine Benutzereingabe gestoppt werden kann.

Die EP 2 123 407 A2 und die EP 2 113 344 A1 zeigen, dass ein Manipulator stillgesetzt wird, wenn der TCP des Manipulators den begrenzten Arbeitsraum verlässt.

Aufgabe der vorliegenden Erfindung ist es daher mit einfacheren Mitteln ein sicheres Einkoppeln und Auskoppeln von Eingabegerät zu Manipulator zu ermöglichen. Überdies soll das System für den Benutzer intuitiv bedienbar sein, und ein möglichst schnelles, sicheres Einkoppeln ermöglichen.

### 3. Ausführliche Beschreibung der Erfindung

Die oben genannten Aufgaben werden gelöst durch ein erfindungsgemäßes Verfahren zum Einkoppeln eines Eingabegerätes mit zumindest einem Manipulator gemäß Anspruch 1, durch ein Verfahren zum Ein - und Auskoppeln einen Eingabegeräts mit zumindest einem Manipulator gemäß Anspruch 7, sowie durch eine entsprechende Steuervorrichtung nach Anspruch 13 und ein maschinenlesbares Medium nach Anspruch 14.

Insbesondere werden die oben genannten Aufgaben gelöst durch ein Verfahren zum Einkoppeln eines Eingabegerätes mit einem Manipulator, wobei das Eingabegerät zur Steuerung des Manipulators eingerichtet ist, und das Verfahren die folgenden Verfahrensschritte umfasst:
a) Prüfen, ob die Pose eines Eingabepunktes des Eingabegeräts innerhalb eines Eingabetoleranzbereichs liegt, wobei der Eingabetoleranzbereich des Eingabegerätes durch eine Abbildung eines Toleranzbereichs eines Bezugspunktes des Manipulators bestimmt ist und der Eingabetoleranzbereich bis zum Einkoppeln des Eingabegeräts mit dem Manipulator unveränderlich ist;
b) Bewegen des Bezugspunktes des Manipulators zu der, durch das Eingabegerät kommandierten Pose, wenn die Pose des Eingabepunkts innerhalb des Eingabetoleranzbereichs liegt;
c) Einkoppeln des Eingabegeräts mit dem Manipulator, nachdem der Bezugspunkt die kommandierte Pose erreicht hat.

Das erfindungsgemäße Verfahren zum Einkoppeln eines Eingabegerätes mit zumindest einem Manipulator ermöglicht ein schnelles und dennoch sicheres Einkoppeln, da es durch den Benutzer nicht notwendig ist, die manuell geführte Pose des Eingabepunktes des Eingabegeräts mit der aktuellen Pose des Bezugspunktes des Manipulators in Übereinstimmung zu bringen. Stattdessen erlaubt das Verfahren, dass der Manipulator eine gewisse, begrenzte Bewegung zu der vom Eingabegerät aktuell kommandierten Pose durchführt. Der Manipulator kommt daher für ein einfaches und schnelles Einkoppeln dem Eingabegerät "entgegen", sobald dieses in einen Eingabetoleranzbereich hineinbewegt wird. Die Bewegung des Manipulators auf die Pose des Eingabegeräts zu, ist dabei durch den Eingabetoleranzbereich und den dazu entsprechenden Toleranzbereich eines Bezugspunktes des Manipulators auf eine sichere Bewegung begrenzt, so dass insbesondere bei laparoskopischen Operationen oder ähnlichen Anwendungsfällen kein Schaden entstehen kann. Somit kann ein Einkoppeln des Eingabegeräts mit dem Manipulator schnell, intuitiv und dennoch sicher erfolgen.

Üblicherweise wird jedem Manipulator ein Eingabegerät zugeordnet, welches sich in translatorischen und rotatorischen Achsen bewegen lässt. Ein Benutzer führt manuell einen Eingabepunkt des Eingabegeräts, wobei die einzelnen Achswerte des Eingabegeräts gemessen werden und daraus die Pose des Eingabepunktes im Raum berechnet wird.

Lageänderungen des Eingabepunktes werden in der Regel unmittelbar in entsprechende Bewegungen des Manipulators umgesetzt. Dabei korrespondiert die Pose des Eingabepunktes mit der Pose eines Bezugspunktes des Manipulators, der relativ zum Manipulator festgelegt ist. Vorzugsweise ist der Bezugspunkt des Manipulators der Tool Center Point (TCP). Erreicht der Manipulator einen Hindernisraum oder die Grenze seines Arbeitsraumes, wird seine Bewegung angehalten und das Eingabegerät von dem Manipulator ausgekoppelt.

Zum erneuten Einkoppeln wird nun um den Bezugspunkt des Manipulators rechnerisch ein Toleranzbereich bestimmt. Dieser Toleranzbereich kann für translatorische Bewegungen eine beliebige Form und Größe annehmen. Form und Größe des Toleranzbereichs werden insbesondere so gewählt, dass eine translatorische Bewegung des Bezugspunktes innerhalb des Toleranzbereichs als sicher angenommen wird. Entsprechend wird der Toleranzbereich in seiner Größe bemessen. Im Hinblick auf rotatorische Bewegungen des Bezugspunktes um seine drei möglichen Raumachsen kann der Toleranzbereich zudem auf bestimmte zulässige Winkelabweichungen beschränkt sein, die der Bezugspunkt des Manipulators zum Einkoppeln rotieren darf. Dann wird der Manipulator sich erst auf die kommandierte Pose zubewegen, wenn einerseits die Position des Eingabepunktes innerhalb der räumlichen Grenzen des Eingabetoleranzbereichs liegt und andererseits die Orientierung des Eingabepunktes innerhalb der vom Toleranzbereich vorgegebenen zulässigen Winkelabweichungen liegt.

Weiterhin kann der Toleranzbereich in der Automatisierungstechnik beispielsweise durch in der Umgebung befindliche Maschinen und Werkstücke begrenzt sein. Bei einer Anwendung in der minimalinvasiven Chirurgie, kann der Toleranzbereich beispielsweise zusätzlich durch in der Umgebung des Bezugspunktes befindliche Risikostrukturen, wie Blutgefäße, Nervenstränge, etc. begrenzt sein.

Der Toleranzbereich des Bezugspunktes des Manipulators wird auf den Eingabebereich des Eingabepunktes des Eingabegeräts abgebildet und so der Eingabetoleranzbereich bestimmt. Die im Eingabetoleranzbereich liegenden Positionen des Eingabepunktes und erlaubten Winkelabweichungen (erlaubte Orientierung des Eingabepunktes) entsprechen somit auf Manipulatorseite den Positionen und erlaubten Winkelabweichungen (Orientierungen) des Bezugspunktes im Toleranzbereich.

Bevorzugt umfasst das Verfahren die folgenden weiteren Verfahrensschritte:
d) Prüfen, ob die Pose des Eingabepunktes innerhalb eines erweiterten Eingabetoleranzbereichs und außerhalb des Eingabetoleranzbereichs liegt, wobei der erweiterte Eingabetoleranzbereich größer ist, als der Eingabetoleranzbereich und wobei der erweiterte Eingabetoleranzbereich den Eingabetoleranzbereich umfasst;
e) Bestimmen einer Annäherungspose des Bezugspunktes, wobei die Annäherungspose innerhalb des Toleranzbereichs des Bezugspunktes liegt, und die Annäherungspose eine Abweichung von der, vom Eingabegerät kommandierten Pose des Bezugspunktes aufweist;
f) Bewegen des Bezugspunktes des Manipulators zu der Annäherungspose, wenn der Eingabepunkt innerhalb des erweiterten Eingabetoleranzbereichs und außerhalb des Eingabetoleranzbereichs liegt; und
g) Einkoppeln des Eingabegeräts mit dem Manipulator, nachdem der Bezugspunkt die Annäherungspose erreicht hat.

Da der Bezugspunkt des Manipulators bei der Bewegung zur Annäherungspose den Toleranzbereich nicht verlässt, ist diese Bewegung sicher.

Der erweiterte Eingabetoleranzbereich ermöglicht ein vereinfachtes Einkoppeln, da der erweiterte Eingabetoleranzbereich größer als der Eingabetoleranzbereich ist, und somit durch den Benutzer leichter gefunden wird. Dieser erweiterte Eingabetoleranzbereich weist für translatorische Bewegungen eine beliebige Form und Größe auf, ist jedoch größer als der räumliche Bereich des Eingabetoleranzbereichs und umfasst den Eingabetoleranzbereich vollständig. Entsprechend umfasst der erweiterte Eingabetoleranzbereich im Hinblick auf rotatorische Bewegungen die zulässige Winkelabweichungen des Eingabetoleranzbereichs vollständig und definiert erweiterte Winkelabweichungen, die eine größer Winkelabweichung als die Winkelabweichungen des Eingabetoleranzbereichs erlauben.

Das Einkoppeln ist auch mit dem erweiterten Eingabetoleranzbereich sicher, da sich der Bezugspunkt des Manipulators lediglich in seinem üblichen Toleranzbereich auf die kommandierte Pose zubewegt. Das Einkoppeln findet dann bereits statt, wenn der Bezugspunkt des Manipulators die Annäherungspose erreicht hat. Es ist somit nicht notwendig, dass der Bezugspunkt des Manipulators die exakt durch das Eingabegerät kommandierte Pose erreicht. Es ändert sich dabei die Abbildung des Eingabepunktes des Eingabegeräts auf den Bezugspunkt des Manipulators. Die Änderung der Abbildung kann alle Achsen des Eingabegeräts betreffen und sowohl translatorisch als auch rotatorisch oder eine beliebige Kombination daraus sein. Der sich ergebende Versatz zwischen kommandierter Pose des Eingabegeräts und Bezugspunkt des Manipulators ist für den Benutzer bei der Bedienung unerheblich und oft auch unmerklich.

Im einfachsten Fall entspricht der erweiterte Eingabetoleranzbereich bevorzugt dem um einen Erweiterungsfaktor skalierten Eingabetoleranzbereich. Jedoch müssen der erweiterte Eingabetoleranzbereich und der Eingabetoleranzbereich nicht konzentrisch sein. Auch die erweiterten Winkelabweichungen müssen für gegenläufige Drehbewegungen nicht gleich sein. Beispielsweise kann ein erweiterter Eingabetoleranzbereich für rotatorische Bewegungen eine erweiterte Winkelabweichung im Uhrzeigersinn um weitere 5° erlauben, für eine rotatorische Bewegung entgegen des Uhrzeigersinns, lediglich um 2°. Es ist auch zulässig, dass Eingabetoleranzbereich und erweiterten Eingabetoleranzbereich einen zumindest teilweise deckungsgleichen Rand oder eine teilweise deckungsgleiche Winkelabweichung aufweisen.

Bevorzugt umfasst das Verfahren weiterhin einen Verfahrensschritt, in dem vor dem Schritt des Bewegens des Bezugspunktes des Manipulators geprüft wird, ob die Pose des Bezugspunkt des Manipulators bei der vom Eingabegerät kommandierten Pose oder der Annäherungspose einen Hindernisraum verletzt. Ein Hindernisraum ist ein Bereich, welcher im technisch möglichen Arbeitsraum des Manipulators liegt, jedoch vom Manipulator nicht angefahren oder durchfahren werden darf. Die zusätzliche Prüfung, ob ein Hindernisraum verletzt wird, erhöht die Sicherheit der Anwendung des Verfahrens. Der Manipulator kann dann beispielsweise gestoppt oder um den Hindernisraum herumgeleitet werden.

Bevorzugt umfasst das Verfahren weiterhin einen Verfahrensschritt, in dem das Bewegen des Bezugspunktes des Manipulators zu der durch das vom Eingabegerät kommandierten Pose in Verfahrensschritt b) abgebrochen wird, wenn der Eingabepunkt den Eingabetoleranzbereich vor Erreichen der kommandierten Pose verlässt und das Bewegen des Bezugspunktes des Manipulators zur Annäherungspose in Verfahrensschritt f) abgebrochen wird, wenn der Eingabepunkt den erweiterten Eingabetoleranzbereich vor Erreichen der Annäherungspose verlässt.

Der Eingabepunkt kann den Eingabetoleranzbereich, als auch den erweiterten Eingabetoleranzbereich durch rotatorische oder translatorische Bewegungen verlassen. Das Abbrechen der Bewegung des Bezugspunktes infolge des Verlassens des Eingabetoleranzbereich oder des erweiterten Eingabetoleranzbereichs des Eingabepunktes verhindert, dass der Manipulator dem Eingabegerät unkontrolliert folgt, ohne dass eine Kopplung stattgefunden hat. Eine Bewegung des Bezugspunktes des Manipulators auf die Kopplungspose zu soll nur zulässig sein, wenn sich Eingabepunkt im Eingabetoleranzbereich oder erweiterten Eingabetoleranzbereich befindet.

Bevorzugt können Form und/oder Größe des Toleranzbereichs, des Eingabetoleranzbereichs und/oder des erweiterten Eingabetoleranzbereichs von zumindest einer weiteren Systemgröße abhängig sein, umfassend die Geschwindigkeit des Manipulators, einen Detektionsbereich, welcher die Detektion der Position und/oder der Orientierung des Bezugspunktes des Manipulators ermöglicht, Kräfte oder Momente, welche am Bezugspunkt des Manipulators wirken, ein aus Umweltdaten bestimmter Hindernisraum, und/oder einer Grenze des Arbeitsraums des Manipulators.

Die Größe des Toleranzbereichs kann an die Umweltbedingungen und Randbedingungen des Manipulatorsystems angepasst werden, um zum Einkoppeln nur sichere Bewegungen zuzulassen.

Bei höheren Geschwindigkeiten des Manipulators kann der Toleranzbereich beispielsweise kleiner bestimmt werden, als bei niedrigeren Geschwindigkeiten.

Innerhalb des Detektionsbereichs kann die Position und/oder Orientierung des Bezugspunktes des Manipulators bestimmt werden. Wird ein Manipulator bei einer minimalinvasiven Operation, wie beispielsweise einer laparoskopischen Operation eingesetzt, kann der Detektionsbereich beispielsweise der Sichtbereich des Endoskops sein. In der Automatisierungstechnik kann der Detektionsbereich beispielsweise der Sichtbereich einer Kamera oder eines anderen Sensorsystems sein. Die Anpassung von Form und Größe des Toleranzbereichs, des Eingabetoleranzbereichs und gegebenenfalls des erweiterten Eingabetoleranzbereichs an den Detektionsbereich ist vorteilhaft, da somit der Manipulator beim Einkoppeln im Detektionsbereich verbleibt und eine neue Ausrichtung des Sensorsystems unterbleiben kann.

Weiterhin können Kräfte und/oder Momente welche am Bezugspunkt des Manipulators wirken die Form und Größe des Toleranzbereichs beeinflussen. Der Toleranzbereich kann demnach bevorzugt dynamisch angepasst werden, wenn eine maximale Kraft oder ein maximales Moment am Bezugspunkt überschritten wird. Dies ist vorteilhaft, wenn ein Schaden erst ab einer bestimmten Kraft bzw. einem bestimmten Moment zu erwarten ist.

Wird ein Manipulator bei einer minimalinvasiven Operation, wie beispielsweise einer laparoskopischen Operation, eingesetzt, so können aus Umweltdaten, beispielsweise aus endoskopischen Bildern, Röntgen-Aufnahmen oder anderen Bilddaten, Hindernisräume ermittelt werden. Auch diese können die Form und Größe des Toleranzbereichs beeinflussen.

Bevorzugt kann das Verfahren weiterhin den folgenden Verfahrensschritt umfassen: Automatisches Bestimmen des erweiterten Eingabetoleranzbereichs, wobei der erweiterte Eingabetoleranzbereich, dem um einen Erweiterungsfaktor skalierten Eingabetoleranzbereich entspricht. Damit kann der erweiterte Eingabetoleranzbereich sehr leicht aus dem Eingabetoleranzbereich berechnet werden. Hierbei kann bevorzugt eine absolute Mindestgröße des erweiterten Eingabetoleranzbereichs vorgegeben sein, um das Koppeln auch bei sehr kleinen Toleranzbereichen zu erleichtern.

Die oben genannten Aufgaben werden weiterhin gelöst durch ein Verfahren zum Ein- und Auskoppeln eines Eingabegerätes mit einem Manipulator, wobei das Eingabegerät zur Steuerung des zumindest einen Manipulators eingerichtet ist und wobei das Verfahren die folgenden Verfahrensschritte umfasst:
a) Auskoppeln des Eingabegeräts von dem Manipulator, wenn der Bezugspunkt des Manipulators einen Hindernisraum oder eine Grenze des Arbeitsraums erreicht;
b) Aktualisieren der Zuordnung der Pose des Eingabepunktes des Eingabegeräts zu der Pose des Bezugspunktes des Manipulators, wobei die aktuelle Pose des Eingabepunktes der letzten erlaubten Pose des Bezugspunktes des Manipulators entspricht;
c) Prüfen, ob die vom Eingabegerät kommandierte Pose des Bezugspunktes des Manipulators in der aktualisierten Zuordnung außerhalb eines Hindernisraums und innerhalb des Arbeitsraums liegt;
d) Einkoppeln des Eingabegeräts mit dem Manipulator, wenn die vom Eingabegerät kommandierte Pose des Bezugspunktes des Manipulators außerhalb des Hindernisraums und innerhalb des Arbeitsraums liegt.

Das Auskoppeln des Eingabegeräts von dem zu mindestens einen Manipulator stellt sicher, dass der Manipulator vom Benutzer nicht in einen Hindernisraum bewegt werden kann, sondern an der Grenze zum Hindernisraum stehen bleibt. Das Erreichen des Hindernisraums und das Erreichen einer Grenze des Arbeitsraums werden vorzugsweise für rotatorische und/oder translatorische Bewegungen ausgewertet. So kann beispielsweise auch sichergestellt werden, dass bei laparoskopischen Operationen das Werkzeug oder das Endoskop stets die Haut oder Bauchdecke des Patienten im gleichen Punkt durchdringt.

Die ständige Aktualisierung der Zuordnung der Pose des Eingabepunktes des Eingabegeräts zu der letzten erlaubten Pose des Bezugspunktes des Manipulators und die anschließende Prüfung, ob die vom Eingabegerät kommandierte Pose des Bezugspunkt des Manipulators nun wieder außerhalb des Hindernisraums und innerhalb des Arbeitsraums liegt, ermöglicht ein Einkoppeln des Eingabegeräts mit dem Manipulator, sobald das Eingabegerät in eine Richtung bewegt wird, die vom Hindernisraum weg weist. Folglich wird aus jeder Stellung des Eingabegeräts heraus eingekoppelt, solange die Bewegung des Eingabegeräts in eine gültige Richtung verläuft, nämlich in eine Richtung die innerhalb des Arbeitsraums vom Hindernisraum weg weist.

Ein weiterer Vorteil des Verfahrens ist, dass sich die Grenze des Hindernisraums des Manipulators automatisch der Grenze des Bewegungsraums des Eingabegeräts entspricht, falls der Benutzer das Eingabegerät nach dem Auskoppeln bis zu einem mechanischen Anschlag in eine ungültige Richtung oder Rotation weiterbewegt. Auf diese Weise kann der Bewegungsraum des Eingabegeräts vollständig zur Telemanipulation des Manipulators ausgenutzt werden.

Bevorzugt erfolgt die Prüfung, ob die vom Eingabegerät kommandierte Pose des Bezugspunktes des Manipulators außerhalb eines Hindernisraums und innerhalb des Arbeitsraumes liegt, in diskreten Zeitschritten. Damit erfolgt eine fortlaufende Abfrage der Stellung des Eingabegeräts.

Bevorzugt ist die Prüfung, ob die vom Eingabegerät kommandierte Pose des Bezugspunktes des Manipulators außerhalb eines Hindernisraums und innerhalb des Arbeitsraums liegt, abhängig von einer Mindeständerung der vom Eingabegerät kommandierten Pose des Bezugspunktes des Manipulators. Eine derartige Mindeständerung ist vorteilhaft, da so ein ungewolltes Ein- und Auskoppeln durch kleinste Bewegungen, wie sie beispielsweise durch einen Tremor des Benutzers oder durch Signalrauschen entstehen könnte, vermieden wird.

Bevorzugt ist die Geschwindigkeit des Manipulators abhängig von zumindest einer Systemgröße, umfassend:
a) einen Detektionsbereichs, welcher die Detektion der Position und/oder Orientierung des Bezugspunktes des Manipulators ermöglicht,
b) Kräfte oder Momente, welche am Bezugspunkte des Manipulators wirken,
c) einen aus Umweltdaten bestimmten Hindernisraum und/oder
d) einer Grenze des Arbeitsraums des Manipulators.

Die Geschwindigkeit des Manipulators ist bevorzugt abhängig von zumindest einer Systemgröße, um eine sichere Bewegung des Manipulators zu erreichen, wobei die Systemgröße einen Detektionsbereich, welcher die Detektion der Position und/oder der Orientierung des Bezugspunktes des Manipulators ermöglicht. Die Geschwindigkeit des Manipulators kann auch von einer Grenze des Arbeitsraums des Manipulators abhängig sein. Die Systemgröße können Kräfte oder Momente sein, welche am Bezugspunkt des Manipulators wirken und/oder sie kann einen aus Umweltdaten bestimmten Hindernisraum umfassen. Damit kann die Geschwindigkeit des Manipulators, insbesondere bestimmt an dessen Bezugspunkt, auf dessen mögliche Bewegung zum Einkoppeln angepasst werden, um ein möglichst schnelles aber sicheres Einkoppeln zu erreichen.

Bevorzugt weist eine Änderung der Pose des Eingabepunktes des Eingabegeräts zu der Änderung des Bezugspunktes des Manipulators ein Übersetzungsverhältnis von i ≥ 1, bevorzugt von i = 2 bis 10, bevorzugter von i = 3 bis 5 auf. Mit diesen Übersetzungsverhältnissen werden insbesondere bei laparoskopischen Operationen größere Bewegungen des Eingabegeräts in kleinere Bewegungen des Manipulators umgesetzt, was feinere Operationsschritte ermöglicht.

Bevorzugt kann das Verfahren weiterhin einen Verfahrensschritt des automatischen Anpassens des Übersetzungsverhältnisses aufweisen. Bevorzugt ist hierbei das Übersetzungsverhältnis von zumindest einer der oben genannten Systemgrößen abhängig.

Das Übersetzungsverhältnis, das die Größe der Änderung der Pose des Eingabepunktes des Eingabegeräts zur Größe der Änderung der Pose des Bezugspunktes des Manipulators angibt, kann durch den Benutzer manuell bestimmt und auch verändert werden. Dies ist insbesondere dann vorteilhaft, wenn der Manipulator in bestimmten Situationen sehr kleine Bewegungen ausführen soll. Weiterhin kann das Übersetzungsverhältnis auch automatisch angepasst werden. Das Übersetzungsverhältnis kann vorzugsweise unabhängig für translatorische und rotatorische Bewegungen angepasst werden. Beispielsweise ist es vorteilhaft, wenn translatorische Bewegungen mit einem Übersetzungsverhältnis i ≥ 1 übersetzt werden, rotatorische Bewegungen hingegen mit einem Übersetzungsverhältnis i = 1 übersetzt werden. Die Anpassung der Übersetzungsverhältnisse ist grundsätzlich bevorzugt auf die jeweilige Anwendung anpassbar. Soll der Manipulator beispielsweise in einem Bereich bewegt werden, welcher von Hindernisräumen umgeben ist die nur einen sehr kleinen Bewegungsspielraum zulassen, so kann das Übersetzungsverhältnis automatisch erhöht werden. Der Benutzer erlangt so eine bessere Kontrolle über den Manipulator.

Bevorzugt gibt das Eingabegerät eine haptische, optische oder akustische Rückmeldung aus, wobei die Rückmeldung des Eingabegeräts das Erreichen eines Hindernisraums des Bezugspunktes und/oder das Einkoppeln oder Auskoppeln des Eingabegeräts mit dem Manipulator und/oder die Entfernung des Eingabepunktes des Eingabegeräts vom Eingabetoleranzbereich und/oder vom erweiterten Eingabetoleranzbereich und/oder von einer korrespondierenden erlaubten Pose des Bezugspunktes des Manipulators anzeigt.

Eine haptische Rückmeldung kann beispielsweise durch Force-Feedback oder Vibrationen des Eingabegerätes erfolgen. Eine optische Rückmeldung kann beispielsweise auf einem Display angezeigt werden oder in Form
von Lichtsignalen, wie beispielsweise mittels LEDs, dem Benutzer angezeigt werden. Eine akustische Rückmeldung kann beispielsweise durch eine Änderung der Tonhöhe, der Lautstärke oder der Frequenz einer Tonfolge erfolgen. Eine Kombination von akustischen, optischen und haptischen Rückmeldungen ist ebenfalls bevorzugt.

Bevorzugt kann die vom Eingabegerät kommandierte Pose des Bezugspunktes des Manipulators dem Benutzer graphisch dargestellt und der tatsächlichen Pose des Bezugspunktes des Manipulators virtuell überlagert werden. Somit kann der Eingebende seine Eingaben des Eingabegerätes graphisch verfolgen, was zu einer Verbesserung der Ergonomie und zu einem schnelleren Einkoppeln führt.

Die oben genannten Aufgaben werden weiterhin gelöst durch eine Steuervorrichtung zum Steuern zumindest eines Manipulators mittels eines Eingabegeräts zur Durchführung des oben beschriebenen Verfahrens, wobei die Steuervorrichtung dazu eingerichtet ist, Eingaben des Eingabegerätes zu empfangen und den Manipulator zu steuern.

Die oben genannten Aufgaben werden weiterhin gelöst durch ein maschinenlesbares Medium, welches Anweisungen speichert, bei deren Ausführung durch eine Steuervorrichtung die Steuervorrichtung einen Manipulator aufgrund Eingaben eines Eingabegeräts gemäß des oben beschriebenen Verfahrens steuert.

### 4. Kurze Beschreibung der Zeichnungen

Im Folgenden werden bevorzugte Ausführungsformen der Erfindung unter Bezugnahme auf die Zeichnungen im Detail erläutert. Hierbei zeigt:
- Fig.1: eine schematische Darstellung eines Manipulatorsystems mit Eingabegerät, Manipulator und Steuereinrichtung;
- Fig. 2: eine schematische Darstellung des Manipulatorsystems nach Fig. 1, mit schematisch eingezeichnetem Toleranzbereich und Eingabetoleranzbereich;
- Fig. 3: eine schematische Darstellung des Manipulatorsystems nach Fig. 2, mit einem schematisch eingezeichneten erweiterten Eingabetoleranzbereich; und
- Figs. 4a - c: schematische Darstellungen des Manipulatorsystems nach Fig. 1 zu unterschiedlichen Zeiten, mit einem schematisch eingezeichnetem Hindernisraum sowie unterschiedlichen Posen des Eingabepunktes des Eingabegeräts und des Bezugspunktes des Manipulators.

### 5. Beschreibung bevorzugter Ausführungsformen

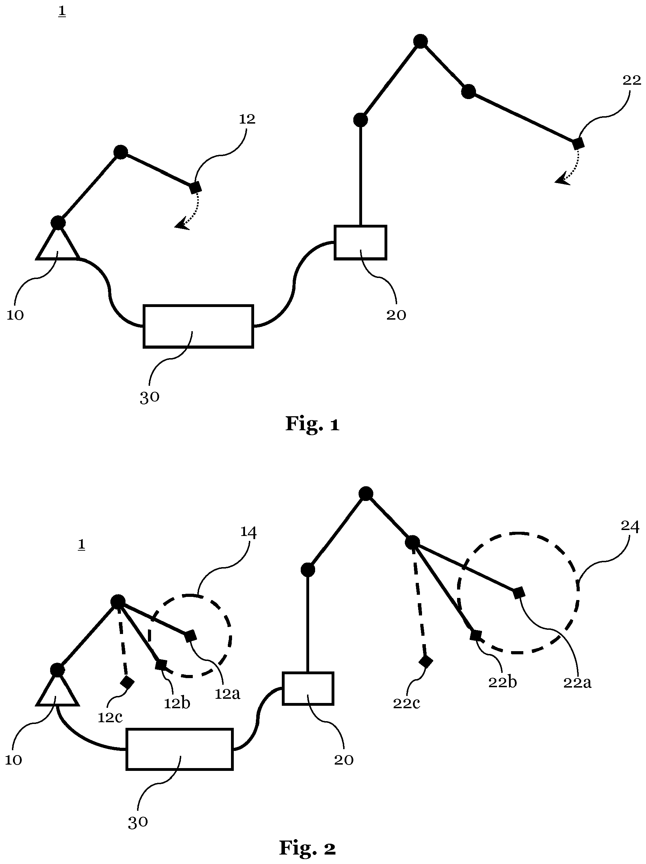

Fig. 1 zeigt ein Manipulatorsystem 1, das ein Eingabegerät 10, einen Manipulator 20 und eine Steuervorrichtung 30 umfasst. Ein Benutzer kann den Manipulator 20 durch manuelles Bewegen des Eingabegeräts 10 fernsteuern (Telemanipulation).

Das Eingabegerät 10 lässt sich bevorzugt in drei translatorischen und drei rotatorischen Achsen bewegen. Ein Benutzer kann so die Position und die Orientierung, also zusammen die Pose, eines, relativ zum Eingabegerät 10 fest definierten Eingabepunktes 12 bestimmen. Zumindest die so definierte Pose oder eine Posenänderung des Eingabepunktes 12 wird an die Steuereinrichtung 30 gesendet und von dieser empfangen. Die Steuereinrichtung 30 setzt die Posenänderung des Eingabepunktes 12 in eine korrespondierende Posenänderung eines Bezugspunktes 22 des Manipulators 20 um. Der Bezugspunkt 22 ist relativ zum Manipulator 20 fest. Bevorzugt ist der Bezugspunkt des Manipulators der Handwurzelpunkt (HWP) des Manipulators oder der Tool-Center-Point (TCP), welcher sich an geeigneter Stelle eines vom Manipulator geführten Objekts befindet. Das Objekt kann beispielsweise ein medizinisches Instrument, ein Werkzeug, ein Werkstück oder ein Sensor sein. Beispielsweise kann der TCP als Spitze eines, vom Manipulator geführten Skalpells definiert sein.

Die Anzahl der Achsen und Glieder von Eingabegerät 10 und Manipulator 20 muss, wie in Fig. 1 dargestellt, nicht übereinstimmen. Weicht die Anzahl der Achsen und Glieder des Manipulators 20 von der Anzahl der Achsen und Glieder des Eingabegeräts 10 ab, so wird eine gültige Stellung des Manipulators berechnet, in welcher die Pose des Bezugspunkt 22 des Manipulators 20 der mittels des Eingabegeräts 10 kommandierten Pose entspricht. Die Stellung des Manipulators ergibt sich aus den einzelnen Achswerten der Achsen des Manipulators.

Fig. 2 zeigt das Manipulatorsystem 1 nach Fig. 1, wobei beispielhaft drei Posen (12a, 12b und 12c) des Eingabepunktes 12 des Eingabegeräts 10 dargestellt sind. Die Posen 12a, 12b und 12c des Eingabepunktes 12 des Eingabegeräts 10 korrespondieren mit drei Posen 22a, 22b und 22c des Bezugspunktes 22 des Manipulators. Um den Bezugspunkt 22 ist ein Toleranzbereich 24 definiert, welcher in Fig. 2 für die translatorischen Bewegungen des Bezugspunktes einer Kugel entspricht, deren Mittelpunkt auf dem Bezugspunkt 22 liegt. Der rotatorische Toleranzbereich, also die erlaubten Winkelabweichungen, ist nicht dargestellt aber vorhanden. Der Toleranzbereich 24 ist so gewählt, dass eine Bewegung des Bezugspunktes 22 des Manipulators 20 innerhalb des Toleranzbereichs 24 als sicher angesehen werden kann. Der Toleranzbereich 24 des Bezugspunkts des 22 wird auf den Eingabebereich des Eingabepunktes 12 des Eingabegeräts 10 abgebildet, so dass sich ein entsprechender Eingabetoleranzbereich 14 ergibt.

In der Pose 12a des Eingabepunktes 12 und der korrespondierenden Pose 22a des Bezugspunktes 22 sei das Eingabegerät 10 vom Manipulator 20 ausgekoppelt worden. Posenänderungen des Eingabepunktes 12 des Eingabegeräts 10 werden in der Folge nicht mehr in Bewegungen des Manipulators 20 umgesetzt. Der Bezugspunkt 22 des Manipulators 20 verharrt in der Pose 22a. Bewegt ein Benutzer den Eingabepunkt 12 des Eingabegeräts 10 im ausgekoppelten Zustand beispielsweise zu der Pose 12c des Eingabepunktes 12, so würde die Pose 22c des Bezugspunktes 22 des Manipulators 20 kommandiert. Die Pose 22c des Bezugspunkt 22 liegt jedoch außerhalb des Toleranzbereichs 24. In diesem Fall ist ein Einkoppeln nicht möglich, da keine sichere selbsttätige Bewegung des Bezugspunktes 22 des Manipulators 20 von der Pose 22a zur Pose 22c sichergestellt werden kann. Folglich bewegt sich der Manipulator 20 nicht und der Bezugspunkt 22 verbleibt in der sicheren Pose 22a.

Bewegt der Benutzer den Eingabepunkt 12 des Eingabegeräts 10 nun beispielsweise in die Pose 12b, welche innerhalb des Eingabetoleranzbereichs 14 liegt, so wird entsprechend die Pose 22b kommandiert. Diese Pose 22b liegt innerhalb des Toleranzbereichs 24 und eine selbsttätige Bewegung des Manipulators 20 zu dieser Pose 22b gilt somit als sicher. Entsprechend erfolgt nun eine Bewegung des Bezugspunktes 22 des Manipulators 20 zur kommandierten Pose 22b. Bevorzugt erfolgt dies aus Sicherheitsgründen mit einer definierten Geschwindigkeit. Der Manipulator 20 bewegt sich daher selbsttätig so, dass sein Bezugspunkt 22 dem Eingabepunkt 12 des Eingabegeräts 10 zur kommandierten Pose 22b entgegen kommt. Erreicht der Bezugspunkt 22 schließlich die Pose 22b, wird das Eingabegerät 10 mit dem Manipulator 20 eingekoppelt. Der Benutzer kann nun den Manipulator 20 durch manuelles Bewegen des Eingabegeräts 10 fernsteuern (Telemanipulation), bis erneut ausgekoppelt wird. Durch das Entgegenkommen des Manipulators 20 zur kommandierten Pose, erfolgt ein Einkoppeln schneller und leichter als bei dem Fall, dass der Benutzer die aktuelle Pose oder Lage des Bezugspunktes 22 Manipulators 20 mit dem Eingabegerät 10 genau treffen muss.

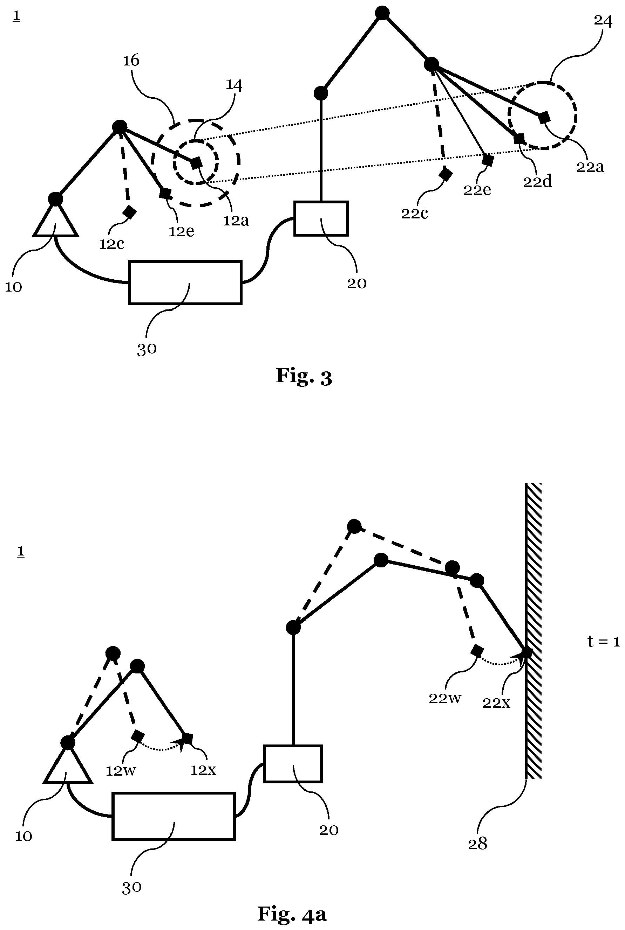

Fig. 3 zeigt das Manipulatorsystem 1 nach Fig. 2, wobei der Eingabetoleranzbereich 14 um einen erweiterten Eingabetoleranzbereich 16 ergänzt ist. Der erweiterte Eingabetoleranzbereich 16 ist für translatorische Bewegungen bevorzugt zum Eingabetoleranzbereich 14 konzentrisch und entspricht bevorzugt einem, um einen Erweiterungsfaktor vergrößerten Eingabetoleranzbereich 14. Der erweiterte Eingabetoleranzbereich für rotatorische Bewegungen, d.h. die erweiterten Winkelabweichungen sind nicht dargestellt, aber vorhanden. In der dargestellten Pose 12a des Eingabepunktes 12 und der korrespondierenden Pose 22a des Bezugspunktes 22 sei das Eingabegerät 10 vom Manipulator 20 ausgekoppelt worden. Während des ausgekoppelten Zustandes wird der Eingabepunkt 12 des Eingabegerätes 10 vom Benutzer beispielsweise in die Pose 12c bewegt. Die Pose 12c des Eingabepunktes 12 entspricht einer kommandierten Pose 22c des Bezugspunkt 22. Die Pose 22c des Bezugspunktes 22 liegt jedoch außerhalb des Toleranzbereichs 24, so dass eine sichere Bewegung des Bezugspunktes 22 von Pose 22a zu der kommandierten Pose 22c nicht sichergestellt werden kann und das oben beschriebene Einkoppeln nicht möglich ist. Zudem liegt die Pose 12c außerhalb des erweiterten Eingabetoleranzbereichs 16. Deshalb verbleibt der Bezugspunkt 22 des Manipulators 20 in der Pose 22a.

Bewegt der Benutzer den Eingabepunkt 12 nun in den erweiterten Eingabetoleranzbereich 16 hinein, hier beispielsweise zur Pose 12e, wird eine Annäherungspose 22d des Bezugspunktes 22 innerhalb des Toleranzbereichs 24 berechnet, die zur kommandierten Pose 22e des Bezugspunktes 22 einen minimalen Abstand aufweist. Eine Bewegung des Bezugspunktes 22 zur Annäherungspose 22d kann als sicher betrachtet werden, da sie innerhalb des Toleranzbereichs 24 liegt. Entsprechend bewegt sich der Manipulator 20 so, dass sein Bezugspunkt 22 zur Annäherungspose 22d bewegt wird.

Erreicht der Bezugspunkt 22 die Annäherungspose 22d, wird das Eingabegerät 10 mit dem Manipulator 20 eingekoppelt und der Pose 12e des Eingabepunktes 12 die Annäherungspose 22d des Bezugspunktes 22 zugeordnet. Im Folgenden wird der Manipulator 20 vom Eingabegerät 10 mit dieser neuen Abbildung angesteuert. Die Abbildung hat nun einen Versatz der der Abweichung von exakt kommandierter Pose 22e des Bezugspunktes 22 zur Annäherungspose 22d entspricht. Der Versatz kann sowohl translatorisch als auch rotatorisch ausgeprägt sein. Bei diesem Einkoppelverfahren kommt der Bezugspunkt 22 des Manipulators 20 dem Eingabepunkt 12 des Eingabegeräts 10 bereits beim Eintritt des Eingabepunktes 12 in den erweiterten Eingabetoleranzbereich 16 so weit entgegen, wie es der Toleranzbereich 24 des Bezugspunktes 22 zulässt und es wird ein Einkoppeln mit verschobener Zuordnung zugelassen. Damit kann ein sicheres Einkoppeln des Eingabegeräts 10 mit dem Manipulator 20 schneller und einfacher erfolgen.

Die Figuren 4a bis 4c zeigen das Manipulatorsystem 1 zu den aufeinanderfolgenden Zeitpunkten t=1, t=2 und t=3. Korrespondierende Posen von Eingabepunkt 12 und Bezugspunkt 22 sind mit den gleichen Buchstaben gekennzeichnet. Beispielsweise entspricht Pose 12w des Eingabepunktes 12 der Pose 22w des Bezugspunktes 22. Die einzelnen Figuren 4a - 4c zeigen jeweils eine in gestrichelten Linien dargestellte Ausgangstellung des Eingabegeräts 10 und des Manipulators 20 und eine in durchgezogenen Linien dargestellte Endstellung des Eingabegeräts 10 und des Manipulators 20. Die in der vorhergehenden Figur dargestellte Endstellung entspricht der, in der nachfolgenden Figur dargestellten Ausgangsstellung. Bewegungen sind schematisch durch Pfeile angedeutet. Zudem zeigen die Figuren 4a bis 4c schematisch einen Hindernisraum 28, welcher vom Bezugspunkt 22 des Manipulators 20 nicht verletzt werden darf.

In Fig. 4a, zum Zeitpunkt t=1 bewegt der Benutzer den Eingabepunkt 12 des Eingabegeräts 10 von der Pose 12w zur Pose 12x und kommandiert so eine Bewegung des Bezugspunktes 22 von der Pose 22w zur Pose 22x. Diese Bewegung ist zulässig, da die Pose 22x des Bezugspunktes 22 den Hindernisraum 28 nicht verletzt. Die Pose 22x liegt gerade an der Grenze des Hindernisraums 28.

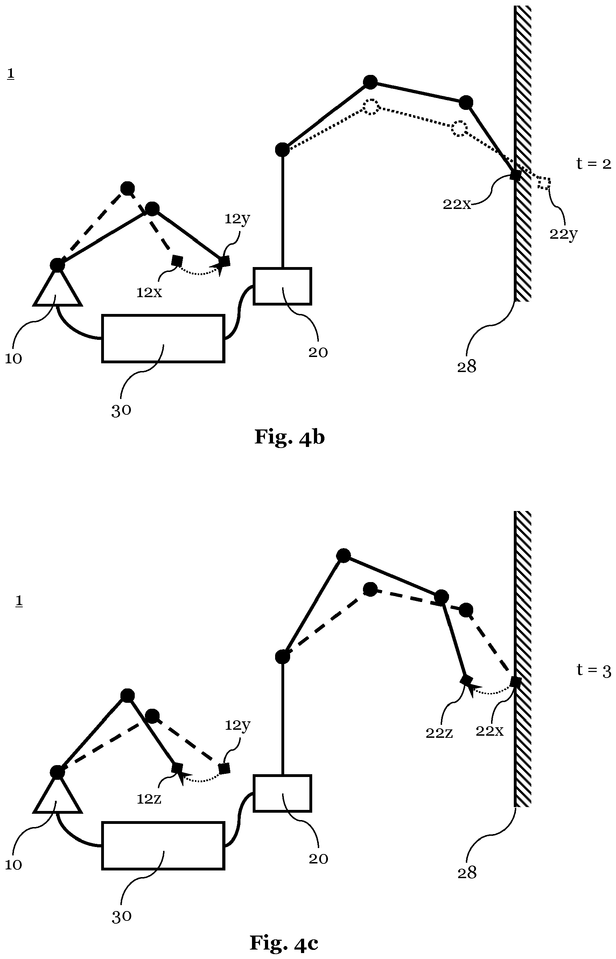

In Fig. 4b, zum Zeitpunkt t=2 bewegt der Benutzer den Eingabepunkt 12 des Eingabegeräts 10 von der Pose 12x zur Pose 12y. Die Pose 12y entspricht einer Pose 22y des Bezugspunktes 22, in welcher der Bezugspunkt 22 den Hindernisraum 28 verletzen würde. Die Stellung des Manipulators 20, welche in der Pose 22y des Bezugspunktes 22 resultieren würde, ist gepunktet dargestellt. Diese potentielle Verletzung des Hindernisraums 28 wird von der Steuereinrichtung 30 erkannt und das Eingabegerät 10 wird vom Manipulator 20 ausgekoppelt. Entsprechend wird die eigentlich kommandierte Bewegung des Bezugspunkts von der Pose 22x zur Pose 22y vom Manipulator 20 nicht durchgeführt. Der Bezugspunkt 22 des Manipulators 20 verbleibt in Pose 22x. Da das Eingabegerät 10 nun vom Manipulator 20 ausgekoppelt ist, kann der Benutzer den Manipulator 20 durch manuelles Bewegen des Eingabegeräts 10 nicht mehr fernsteuern.

Das Eingabegerät 10 kann jedoch auch im ausgekoppelten Zustand frei bewegt werden. In festen Zeitintervallen wird die Pose des Eingabepunktes 12 erfasst, und zunächst überprüft, ob die kommandierte Pose des Bezugspunktes 22 innerhalb oder außerhalb des Hindernisraums 28 liegt. Im in Fig. 4b gezeigten Fall, wurde der Eingabepunkt 12 in die Pose 12y bewegt. Da die korrespondierende Pose 22y, wie oben beschrieben innerhalb des Hindernisraums 28 liegt und nicht angefahren werden kann, wird nun der Pose 12y des Eingabepunktes 12 die letzte erlaubte Pose, hier die Pose 22x des Bezugspunktes 22 zugeordnet. Folglich entspricht die aktuelle Pose des Eingabepunktes 12 nach der Aktualisierung der Zuordnung immer der letzten erlauben Pose des Bezugspunkts 22.

In Fig. 4 c, zum Zeitpunkt t=3 bewegt der Benutzer den Eingabepunkt 12 des Eingabegeräts 10 von der Pose 12y zur Pose 12z. Da der Pose 12y die letzte erlaubte Pose 22x des Bezugspunktes 22 zugeordnet ist, entspricht die neue kommandierte Pose 22z des Bezugspunktes 22 einer Pose, welche außerhalb des Hindernisraums 28 liegt. Folglich kann zum Zeitpunkt t=3 das Eingabegerät 10 mit dem Manipulator 20 eingekoppelt werden und das Eingabegerät 10 den Manipulator 20 mit einer geänderten Zuordnung fernsteuern. Durch das ständige Aktualisieren der Zuordnung der Pose des Eingabepunktes 12 zu der letzten erlaubten Pose des Bezugspunktes 22, kann das Eingabegerät 10 mit dem Manipulator 20 eingekoppelt werden, sobald der Eingabepunkt 12 in einer Richtung oder Rotation bewegt wird, die vom Hindernisraum 28 weg weist. Somit ist ein schnelleres Einkoppeln möglich. Bei diesem Einkoppelverfahren ändert sich daher ständig die Zuordnung von Eingabegerät 10 und Manipulator 20 im ausgekoppelten Zustand bis sie beim Einkoppeln festgelegt wird.

Die oben beschriebenen translatorischen Bewegungen dienen lediglich der einfacheren Darstellung der Einkoppelverfahren. Der Manipulator kann jedoch ebenso rotatorische Bewegungen und/oder Kombinationen von translatorischen und rotatorischen Bewegungen ausführen und wobei das bevorzugte Verfahren zum Einkoppeln auf entsprechende Weise angewendet werden kann.

### Bezugszeichenliste:

- 1: Manipulatorsystem
- 10: Eingabegerät
- 12: Eingabepunkt
- 12 a, b, c, e w, x, y, z: Posen des Eingabepunktes 12
- 14: Eingabetoleranzbereich
- 16: Erweiterter Eingabetoleranzbereich
- 20: Manipulator
- 22: Bezugspunkt
- 22 a, b, c, d, e, w, x, y, z: Posen des Bezugspunktes 22
- 24: Toleranzbereich des Bezugspunktes 22
- 28: Hindernisraum
- 30: Steuervorrichtung

## Patentansprüche

1. Verfahren zum Einkoppeln, nämlich ein Zulassen, insbesondere ein Wieder-Zulassen, einer Umsetzung von Benutzereingaben in Manipulatorbewegungen, eines Eingabegerätes (10) mit einem Manipulator (20), wobei das Eingabegerät (10) zur Steuerung des Manipulators (20) eingerichtet ist, und das Verfahren die folgenden Verfahrensschritte umfasst:
a) Prüfen, ob die Pose, nämlich die Position und Orientierung, des Eingabepunktes (12) des Eingabegeräts (10) innerhalb eines Eingabetoleranzbereichs (14) liegt, wobei der Eingabetoleranzbereich (14) des Eingabegerätes (10) durch eine Abbildung eines Toleranzbereichs (24) eines Bezugspunktes (22) des Manipulators (20) bestimmt ist, und der Eingabetoleranzbereich (14) bis zum Einkoppeln des Eingabegeräts (10) mit dem Manipulator (20) unveränderlich ist;
b) Bewegen des Bezugspunktes (22) des Manipulators (20) zu der, durch das Eingabegerät (10) kommandierten Pose (22b), wenn die Pose des Eingabepunktes (12) innerhalb des Eingabetoleranzbereichs (14) liegt;
c) Einkoppeln des Eingabegeräts (10) mit dem Manipulator (20), nachdem der Bezugspunkt (22) die kommandierte Pose (22b) erreicht hat.

2. Verfahren nach Anspruch 1, weiter umfassend die folgenden Verfahrensschritte:
d) Prüfen, ob die Pose des Eingabepunktes (12) innerhalb eines erweiterten Eingabetoleranzbereichs (16) und außerhalb des Eingabetoleranzbereichs (14) liegt, wobei der erweiterte Eingabetoleranzbereich (16) größer ist, als der Eingabetoleranzbereich (14) und wobei der erweiterte Eingabetoleranzbereich (16) den Eingabetoleranzbereich (14) umfasst;
e) Bestimmen einer Annäherungspose (22d) des Bezugspunktes (22), wobei die Annäherungspose (22d) innerhalb des Toleranzbereichs (24) des Bezugspunktes (22) liegt, und die Annäherungspose (22d) eine Abweichung von der, vom Eingabegerät (10) kommandierten Pose (22b) des Bezugspunktes (22) aufweist;
f) Bewegen des Bezugspunktes (22) des Manipulators (20) zu der Annäherungspose (22d), wenn die Pose des Eingabepunktes (12) innerhalb des erweiterten Eingabetoleranzbereichs (16) und außerhalb des Eingabetoleranzbereichs (14) liegt;
g) Einkoppeln des Eingabegeräts (10) mit dem zumindest einem Manipulator (20), nachdem der Bezugspunkt (22) die Annäherungspose (22d) erreicht hat.

3. Verfahren nach einem der Ansprüche 1 oder 2, wobei das Verfahren weiterhin vor dem Schritt des Bewegens des Bezugspunktes (22) des Manipulators (20) den folgenden Verfahrensschritt umfasst:
h) Prüfen, ob die Pose des Bezugspunktes (22) des Manipulators (22) bei der vom Eingabegerät kommandierten Pose (22b) oder der Annäherungspose (22d) einen Hindernisraum (28) verletzt.

4. Verfahren nach einem der vorhergehenden Ansprüche, wobei das Bewegen des Bezugspunktes (22) des Manipulators (20) zu der durch das Eingabegerät (10) kommandierten Pose (22b) in Verfahrensschritt b) abgebrochen wird, wenn der Eingabepunkt (12) den Eingabetoleranzbereich (14) vor Erreichen der kommandierten Pose (22b) verlässt, und wobei
das Bewegen des Bezugspunktes (22) des Manipulators (20) zu der Annäherungspose (22d) in Verfahrensschritt f) abgebrochen wird, wenn der Eingabepunkt (12) den erweiterten Eingabetoleranzbereich (16) vor Erreichen der Annäherungspose (22d) verlässt.

5. Verfahren nach einem der vorhergehenden Ansprüche, wobei Form und/oder Größe des Toleranzbereichs (24), des Eingabetoleranzbereichs (14) und/oder des erweiterten Eingabetoleranzbereichs (16) abhängig sind von zumindest einer weiteren Systemgröße, umfassend
a) die Geschwindigkeit des Manipulators (20),
b) ein Detektionsbereich, welcher die Detektion der Position und/oder der Orientierung des Bezugspunktes (22) des Manipulators (20) ermöglicht,
c) Kräfte oder Momente, welche am Bezugspunkte (22) des Manipulators (20) wirken,
d) ein aus Umweltdaten bestimmter Hindernisraum (28) und/oder
e) einer Grenze des Arbeitsraums des Manipulators.

6. Verfahren nach einem der Ansprüche 2 bis 5, wobei das Verfahren weiterhin den folgenden Verfahrensschritt umfasst:
- Automatisches Bestimmen des erweiterten Eingabetoleranzbereichs (16), wobei der erweiterte Eingabetoleranzbereich (16), dem um einen Erweiterungsfaktor skalierten Eingabetoleranzbereich (14) entspricht.

7. Verfahren zum Ein- und Auskoppeln eines Eingabegerätes (10) mit einem Manipulator (20), wobei das Eingabegerät (10) zur Steuerung des Manipulators (20) eingerichtet ist, und das Verfahren die folgenden Verfahrensschritte umfasst:
a) Auskoppeln, nämlich eine Unterbrechung der Umsetzung von Benutzereingaben in Manipulatorbewegungen, des Eingabegeräts (10) von dem Manipulator (20), wenn der Bezugspunkt (22) des Manipulators (20) einen Hindernisraum (28) oder eine Grenze des Arbeitsraums erreicht;
b) Aktualisieren der Zuordnung der Pose des Eingabepunktes (12) des Eingabegeräts (10) zu der Pose des Bezugspunktes (22) des Manipulators (20), wobei die aktuelle Pose des Eingabepunktes (12y) zu der letzten erlaubten Pose des Bezugspunktes (22x) des Manipulators (20) zugeordnet wird,
c) Prüfen, ob die vom Eingabegerät (10) kommandierte Pose (22y; 22z) des Bezugspunktes (22) des Manipulators (20) in der aktualisierten Zuordnung außerhalb des Hindernisraums (28) und innerhalb des Arbeitsraums liegt;
d) Einkoppeln des Eingabegeräts (10) mit dem Manipulator (20), wenn die vom Eingabegerät (10) kommandierte Pose (22y; 22z) des Bezugspunktes (22) des Manipulators (20) außerhalb des Hindernisraums (28) und innerhalb des Arbeitsraums liegt.

8. Verfahren nach Anspruch 7, wobei die Prüfung, ob die vom Eingabegerät (10) kommandierte Pose (22y; 22z) des Bezugspunktes (22) des Manipulators (20) außerhalb eines Hindernisraums (28) und innerhalb des Arbeitsraums liegt, in diskreten Zeitschritten erfolgt.

9. Verfahren nach einem der Ansprüche 7 oder 8, wobei die Prüfung, ob die vom Eingabegerät (10) kommandierte Pose (22y; 22z) des Bezugspunktes (22) des Manipulators (20) außerhalb des Hindernisraums (28) und innerhalb des Arbeitsraums liegt, abhängig ist von einer Mindeständerung der vom Eingabegerät (10) kommandierten Pose des Bezugspunktes (22) des Manipulators (20).

10. Verfahren nach einem der vorhergehenden Ansprüche, wobei die Geschwindigkeit des Manipulators (20) abhängig ist von zumindest einer Systemgröße, umfassend:
a) einen Detektionsbereichs, welcher die Detektion der Position und/oder Orientierung des Bezugspunktes (22) des Manipulators (20) ermöglicht;
b) Kräfte oder Momente, welche am Bezugspunkt (22) des Manipulators (20) wirken; und/oder
c) einen aus Umweltdaten bestimmten Hindernisraum (28)
d) einer Grenze des Arbeitsraums des Manipulators (20).

11. Verfahren nach einem der vorhergehenden Ansprüche, wobei eine Änderung der Pose des Eingabepunktes (12) des Eingabegeräts (10) zu der Änderung der Pose des Bezugspunktes (22) des Manipulators (20) ein Übersetzungsverhältnis von i ≥ 1 oder von i = 2 bis 10, oder von i = 3 bis 5 aufweist.

12. Verfahren nach einem der vorhergehenden Ansprüche, weiterhin aufweisend den Schritt des Ausgebens einer haptischen, optischen oder akustischen Rückmeldung, wobei
die Rückmeldung das Erreichen eines Hindernisraums (28) des Bezugspunktes (22), und/oder
das Einkoppeln oder Auskoppeln des Eingabegeräts (10) mit dem Manipulator (20), und/oder
die Entfernung des Eingabepunktes (12) des Eingabegeräts (10) vom Eingabetoleranzbereich (14) und/oder vom erweiterten Eingabetoleranzbereich (16) und/oder von einer korrespondierenden erlaubten Pose des Bezugspunktes (22) des Manipulators (20) anzeigt.

13. Steuervorrichtung (30), zur Steuerung zumindest eines Manipulators (20) mittels eines Eingabegeräts (10) zur Durchführung des Verfahrens gemäß den Ansprüchen 1 bis 13, wobei die Steuervorrichtung dazu eingerichtet ist, Eingaben des Eingabegerätes (10) zu empfangen und den Manipulator (20) zu steuern.

14. Maschinenlesbares Medium, welches Anweisungen speichert, bei deren Ausführung durch eine Steuervorrichtung (30) die Steuervorrichtung (30) einen Manipulator (20) aufgrund Eingaben eines Eingabegeräts (10) gemäß einem Verfahren nach den Ansprüchen 1 bis 13 steuert.

## Claims

1. A method for coupling, i.e. allowing, in particular re-allowing a conversion of user input into manipulator movements, of an input device to a manipulator (20), wherein the input device (10) is configured to control the manipulator (20), the method comprising the following steps:
a) checking whether the pose, i.e. the position and orientation of an input point (12) of the input device (10) is within an input tolerance range (14), wherein the input tolerance range (14) of the input device (10) is determined by mapping a tolerance range (24) of a reference point (22) of the manipulator (20), and the input tolerance range (14) is unchangeable until the input device (10) is coupled to the manipulator (20);
b) moving the reference point (22) of the manipulator (20) to the pose commanded (22b) by the input device (10) when the pose of the input point (12) is within the input tolerance range (14); and
c) coupling the input device (10) to the manipulator (20) after the reference point (22) has reached the commanded pose (22b).

2. The method of claim 1, further comprising the following steps:
d) checking whether the pose of the input point (12) is within an extended input tolerance range (16) and outside the input tolerance range (14), wherein the extended input tolerance range (16) is greater than the input tolerance range (14), and wherein the extended input tolerance range (16) comprises the input tolerance range (14);
e) determining an approach pose (22d) of the reference point (22), wherein the approach pose (22d) is within the tolerance range (24) of the reference point (22), and the approach pose (22d) has a minimum deviation from the pose (22b) of the reference point that is commanded by the input device (10);
f) moving the reference point (22) of the manipulator to the approach pose (22d) when the pose of the input point (12) is within the extended input tolerance range (16) and outside the input tolerance range (14); and
g) coupling the input device (10) to the at least one manipulator (20) after the reference point (22) has reached the approach pose (22d).

3. The method of claim 1 or 2, wherein, prior to the step of moving the reference point (22) of the manipulator (10), the method further comprises the following step:
h) checking whether the pose of the reference point (22) of the manipulator (20) violates an obstacle space (28) in the input device-commanded pose (22b) or the approach pose (22d).

4. The method of any of the preceding claims,
wherein the moving the reference point (22) of the manipulator (20) to the input device-commanded pose (22b) in method step b) is terminated, when the input point (12) leaves the input tolerance range (14) before reaching the commanded pose (22b); and
wherein the moving the reference point (22) of the manipulator (20) to the approach pose (22d) in method step f) is terminated, when the input point (12) leaves the extended input tolerance range (16) before reaching the approach pose (22d).

5. The method of any of the preceding claims, wherein shape and/or size of the tolerance range (24), of the input tolerance range (14), and / or of the extended input tolerance range (16) is dependent on at least one other system variable, wherein the at least one other system variable comprises:
a) the speed of the manipulator (20)
b) a detection range that enables the detection of at least one of the position or orientation of the reference point (22) of the manipulator (20),
c) forces or torques that act on the reference point (22) of the manipulator (20),
d) an obstacle space (28) determined from environmental data, and / or
e) a boundary of a working space of the manipulator.

6. The method of any of the claims 2 - 5, further comprising the following step:
automatically determining the extended input tolerance range (16), wherein the extended input tolerance range (16) corresponds to the input tolerance range (14) scaled by an extension factor.

7. A method for coupling and decoupling an input device (10) to and from a manipulator (20), wherein the input device (10) is configured to control the manipulator (20), the method comprising the following steps:
a) decoupling, i.e. interrupting the conversion of user input into manipulator movements, the input device (10) from the manipulator (20) when the reference point (22) of the manipulator (20) has reached an obstacle space (28) or a boundary of a working space;
b) updating a mapping of the pose of an input point (12) of the input device (10) to the pose of the reference point (22) of the manipulator (20), wherein a current pose of the input point (12y) is mapped to the last allowed pose of the reference point (22x) of the manipulator (20);
c) checking whether the input device-commanded pose (22y, 22z) of the reference point (22) of the manipulator (20) in the updated mapping is outside the obstacle space (28) and inside the working space; and
d) coupling the input device (10) to the manipulator (20) when the input device (10) -commanded pose (22y, 22z) of the reference point (22) of the manipulator (20) is outside the obstacle space (28) and inside the working space.

8. The method of claim 7, wherein the checking whether the input device (10) - commanded pose (22y, 22z) of the reference point (22) of the manipulator (20) is outside an obstacle space (28) and inside the working space takes place in discrete time steps.

9. The method of claim 7 or 8, wherein the checking whether the input device (10) - commanded pose (22y, 22z) of the reference point (22) of the manipulator (20) is outside the obstacle space (28) and inside the working space is dependent on a minimum change in the input device (10) -commanded pose of the reference point (22) of the manipulator (20).

10. The method of any of the preceding claims, wherein the speed of the manipulator (10) is dependent on at least one system variable, the at least one system variable comprising at least one of:
a) a detection range that enables the detection of the position and / or orientation of the reference point (22) of the manipulator (20);
b) forces or torques that act on the reference point (22) of the manipulator (20); and / or
c) an obstacle space (28) determined from environmental data;
d) a boundary of the working space of the manipulator (20).

11. The method of any of the preceding claims, wherein a change in the pose of the input point (12) of the input device (10) to the change of the pose of the reference point (22) of the manipulator (20) has a translation ratio of i ≥ 1, or of i= 2 to 10, or of i = 3 to 5.

12. The method of any of the preceding claims, further comprising;
outputting a haptic, visual, or audible feedback, wherein the feedback indicates at least one of:
that an obstacle space (28) of the reference point (22) has been reached;
that the input device (10) has been coupled to or decoupled from the manipulator (20);
the distance of the input point (12) of the input device (10) from the input tolerance range (14) and / or form the extended input tolerance range (16) and / or form a corresponding allowed pose of the reference point (22) of the manipulator (20).

13. A control device (30) for controlling at least one manipulator (20) with an input device (10) to carry out the method of any of the preceding claims 1 to 13, wherein the control device is configured to receive inputs from the input device (10) and to control the manipulator (20).

14. Machine-readable medium, which stores instructions, in the course of execution of which by a control device (30) the control device (30) controls a manipulator (20) on the basis of inputs of an input device (10) according to a method according to claims 1 to 13.

## Revendications

1. Procédé de couplage, à savoir d'autorisation, en particulier de ré-autorisation, d'une conversion d'entrées utilisateur en mouvements de manipulateur, d'un appareil d'entrée (10) à un manipulateur (20), l'appareil d'entrée (10) étant agencé pour commander le manipulateur (20), et le procédé comprenant les étapes de procédé suivantes :
a) vérification que la pose, à savoir la position et l'orientation, du point d'entrée (12) de l'appareil d'entrée (10) se trouve à l'intérieur de la zone de tolérance d'entrée (14), la zone de tolérance d'entrée (14) de l'appareil d'entrée (10) étant déterminée par une représentation d'une zone de tolérance (24) du point de référence (22) du manipulateur (20), et la zone de tolérance d'entrée (14) étant invariable jusqu'au couplage de l'appareil d'entrée (10) avec le manipulateur (20) ;
b) déplacement du point de référence (22) du manipulateur (20) jusqu'à la pose (22b) commandée par l'intermédiaire de l'appareil d'entrée (10) si la pose du point d'entrée (12) se trouve à l'intérieur de la zone de tolérance d'entrée (14) ;
c) couplage de l'appareil d'entrée (10) au manipulateur (20) après que le point de référence (22) a atteint la pose commandée (22b).

2. Procédé selon la revendication 1, comprenant également les étapes de procédé suivantes :
d) vérification que la pose du point d'entrée (12) se trouve à l'intérieur d'une zone de tolérance d'entrée élargie (16) et à l'extérieur de la zone de tolérance d'entrée (14), la zone de tolérance d'entrée élargie (16) étant plus grande que la zone de tolérance d'entrée (14), et la zone de tolérance d'entrée élargie (16) englobant la zone de tolérance d'entrée (14) ;
e) détermination d'une pose approximative (22d) du point de référence (22), la pose approximative (22d) se trouvant à l'intérieur de la zone de tolérance (24) du point de référence (22), et la pose approximative (22d) présentant un écart par rapport à la pose (22b) du point de référence (22) commandée par l'appareil d'entrée (10) ;
f) déplacement du point de référence (22) du manipulateur (20) jusqu'à la pose approximative (22d) si la pose du point d'entrée (12) se trouve à l'intérieur de la zone de tolérance d'entrée élargie (16) et à l'extérieur de la zone de tolérance d'entrée (14) ;
g) couplage de l'appareil d'entrée (10) au au moins un manipulateur (20) après que le point de référence (22) a atteint la pose approximative (22d) .

3. Procédé selon une des revendications 1 ou 2, le procédé comprenant également l'étape de procédé suivante avant l'étape de déplacement du point de référence (22) du manipulateur (20) :
h) vérification que la pose du point de référence (22) du manipulateur (22) ne viole pas un espace d'obstacle (28) dans le cas de la pose (22b) commandée par l'appareil d'entrée ou de la pose approximative (22d).

4. Procédé selon une des revendications précédentes, le déplacement du point de référence (22) du manipulateur (20) jusqu'à la pose (22b) commandée par l'appareil d'entrée (10) à l'étape de procédé b) étant interrompu si le point d'entrée (12) quitte la zone de tolérance d'entrée (14) avant l'atteinte de la pose commandée (22b), et
le déplacement du point de référence (22) du manipulateur (20) jusqu'à la pose approximative (22d) à l'étape de procédé f) étant interrompu si le point d'entrée (12) quitte la zone de tolérance d'entrée élargie (16) avant l'atteinte de la pose approximative (22d).

5. Procédé selon une des revendications précédentes, la forme et/ou la taille de la zone de tolérance (24), de la zone de tolérance d'entrée (14) et/ou de la zone de tolérance d'entrée élargie (16) étant dépendantes d'au moins une grandeur de système supplémentaire, comprenant
a) la vitesse du manipulateur (20),
b) une zone de détection qui permet la détection de la position et/ou de l'orientation du point de référence (22) du manipulateur (20),
c) des forces ou des moments qui agissent au point de référence (22) du manipulateur (20),
d) un espace d'obstacle (28) déterminé à partir de données ambiantes et/ou
e) une limite de l'espace de travail du manipulateur.

6. Procédé selon une des revendications 2 à 5, le procédé comprenant en outre l'étape de procédé suivante :
- détermination automatique de la zone de tolérance d'entrée élargie (16), la zone de tolérance d'entrée élargie (16) correspondant à la zone de tolérance d'entrée (14) modifiée selon un facteur d'élargissement.

7. Procédé de couplage et de découplage d'un appareil d'entrée (10) à un manipulateur (20), l'appareil d'entrée (10) étant agencé pour commander le manipulateur (20), et le procédé comprenant les étapes de procédé suivantes :
a) découplage, à savoir une interruption de la conversion d'entrées utilisateur en mouvements de manipulateur, de l'appareil d'entrée (10) par rapport au manipulateur (20) si le point de référence (22) du manipulateur (20) atteint un espace d'obstacle (28) ou une limite de l'espace de travail ;
b) actualisation de l'association de la pose du point d'entrée (12) de l'appareil d'entrée (10) à la pose du point de référence (22) du manipulateur (20), la pose instantanée du point d'entrée (12y) étant associée à la dernière pose autorisée du point de référence (22x) du manipulateur (20),
c) vérification que, dans l'association actualisée, la pose (22y ; 22z) du point de référence (22) du manipulateur (20) commandée par l'appareil d'entrée (10) se trouve à l'extérieur de l'espace d'obstacle (28) et à l'intérieur de l'espace de travail ;
d) couplage de l'appareil d'entrée (10) au manipulateur (20) si la pose (22y ; 22z) du point de référence (22) du manipulateur (20) commandée par l'appareil d'entrée (10) se trouve à l'extérieur de l'espace d'obstacle (28) et à l'intérieur de l'espace de travail.

8. Procédé selon la revendication 7, la vérification que la pose (22y ; 22z) du point de référence (22) du manipulateur (20) commandée par l'appareil d'entrée (10) se trouve à l'extérieur d'un espace d'obstacle (28) et à l'intérieur de l'espace de travail s'effectuant dans des pas de temps discrets.

9. Procédé selon une des revendications 7 ou 8, la vérification que la pose (22y ; 22z) du point de référence (22) du manipulateur (20) commandée par l'appareil d'entrée (10) se trouve à l'extérieur de l'espace d'obstacle (28) et à l'intérieur de l'espace de travail étant dépendante d'une variation minimale de la pose du point de référence (22) du manipulateur (20) commandée par l'appareil d'entrée (10).

10. Procédé selon une des revendications précédentes, la vitesse du manipulateur (20) étant dépendante d'au moins une grandeur de système, comprenant :
a) une zone de détection qui permet la détection de la position et/ou de l'orientation du point de référence (22) du manipulateur (20) ;
b) des forces ou des moments qui agissent au point de référence (22) du manipulateur (20) ; et/ou
c) un espace d'obstacle (28) déterminé à partir de données ambiantes (28)
d) une limite de l'espace de travail du manipulateur (20) .

11. Procédé selon une des revendications précédentes, une variation de la pose du point d'entrée (12) de l'appareil d'entrée (10) présentant avec la variation de la pose du point de référence (22) du manipulateur (20) un rapport de multiplication de i ≥ 1 ou de i = 2 à 10, ou de i = 3 à 5.

12. Procédé selon une des revendications précédentes, comprenant en outre l'étape d'émission d'un retour haptique, optique ou acoustique,
le retour indiquant l'atteinte d'un espace d'obstacle (28) du point de référence (22), et/ou
le couplage ou le découplage de l'appareil d'entrée (10) au manipulateur (20), et/ou
l'éloignement du point d'entrée (12) de l'appareil d'entrée (10) par rapport à la zone de tolérance d'entrée (14) et/ou à la zone de tolérance d'entrée élargie (16) et/ou à une pose autorisée correspondante du point de référence (22) du manipulateur (20).

13. Dispositif de commande (30) pour la commande d'au moins un manipulateur (20) au moyen d'un appareil d'entrée (10) pour mettre en oeuvre un procédé selon les revendications 1 à 13, le dispositif de commande étant agencé pour recevoir des entrées de l'appareil d'entrée (10) et pour commander le manipulateur (20).

14. Moyen lisible par machine, lequel mémorise des instructions lors de l'exécution desquelles par un dispositif de commande (30) le dispositif de commande (30) commande un manipulateur (20) sur la base d'entrées d'un appareil d'entrée (10) conformément à un procédé selon les revendications 1 à 13.
